# EUROPEAN PATENT APPLICATION

(11) **EP 1 878 428 A1**
(43) Date of publication of application: **16.01.2008**
(21) Application number: 06722303.2
(22) Date of filing: 11.04.2006
(51) Int. Cl.: A61K 31/4412, A61K 31/4418, A61P 43/00

(54) **1-(SUBSTITUTED PHENYL)-5- METHYL- 2 - (1H) PYRIDONE IN THE MANUFACTURE OF MEDICAMENTS FOR TREATING FIBROSIS IN ORGANS OR TISSUES**

(30) Priority: 13.04.2005 CN 200510031445
(71) Applicant: Xiangya Hospital of Central South University, Hunan 410008 (CN)
(72) Inventor: TAO, Lijian, Hunan 410008 (CN); HU, Gaoyun, Hunan 410008 (CN)
(74) Representative: Grättinger & Partner (GbR)
(86) International application number: PCT/CN2006/000651
(87) International publication number: WO 2006/108354

(57) **Abstract**

The use of 1-(substituted phenyl)-5-methyl-2-(1H) pyridone in the manufacture of medicaments for treating fibrosis in organs or tissues. It indicates that 1-(substituted phenyl)-5-methyl-2-(1H) pyridone can inhibit the cells produced by various ECM, and their effect is higher than pirfenidone. Therefore, this tpye of compounds can be used as active ingredient for preparing the antifibrotic agent in organs and tissues.

## Description

### Abstract

The use of 5-METHYL-1-(SUBSTITUTED PHENYL)-2-(1H)-PYRIDONE in the manufacturing of medicaments for treating fibrosis in organs or tissues. It indicates that 5 methyl-1-(substituted phenyl)-2(1H) pyridone can inhibit the cells produced by various ECM, and their effect is higher than pirfenidone. Therefore, this type of compounds can be used as active ingredient for preparing the antifibrotic agent in organs and tissues.

### Field of the Invention

The present invention relates to the use of 5-methyl-1-(substituted phenyl)-2(1H) pyridones. More particularly, the present invetion relates to the use of 5-methyl-1-(substituted phenyl)-2(1H) pyridones in the manufacturing of medicaments for treating fibrosis in organs or tissues.

### Background of the Invention

Fibrosis can occur in various organs or tissues, causing reduction of healthy cells within any organ or tissue and increase in the mass of fibrotic connective tissues, eventually damage the normal structure of the organs or tissues. The damages can impair the physiological and biochemical functions of the affected organs or tissues, and may cause organ shut down completely. The pathogenesis, diagnostic methods, methods of prevention and treatment for organ and tissue fibrosis have been studied extensively. Much progress has been made in certain areas. However there are still many challenges, especially in the area of developing effective therapeutics.

It is generally believed that fibrosis of organs or tissues are caused by multiple factors such as inflammation, immunological reactions, ischemia, hemodynamics change etc. that cause inflammatory denaturing and narcosis of parenchymal cells. The impaired parenchymal cells in turn activate macrophages to release numerous cytokines and growth factors, among which TGF-β is a critical one. TGF-β can activate quiescent extracellular matrix (ECM) producing cells and turn them into myofibroblast. The newly formed fibroblasts not only increase production of collagen, a key protein of ECM, but also decrease destruction of ECM. The net result is accumulation of extracellular matrix that leads to organ or tissue fibrosis. Thus, initiation and development of organ or tissue fibrosis is the results of inflammatory response and production of inflammatory cytokines, mainly TGF-β. Due to the crucial role of TGF-β on ECM accumulation and formation of organ or tissue fibrosis, logically, one of the important goals in early development of antifibrotic drugs is to search compound, which can inhibit the production of pro-inflammatory cytokine, TGF-β. However, more convincing data for any antifibrotic drug candidate will obviously come from the test using various *in vivo* fibrotic models.

A number of compounds that exhibit anti-inflammatory and anti-fibrotic activities have been described. U.S. patents 3,839,346, 3,974,281, 4,042,699 and 4,052,509 published a total of 29 compounds with the following pyridone-like formula (0): 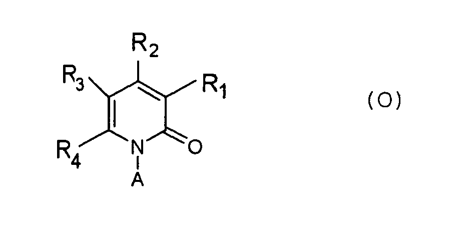 wherein A is an aromatic group. These compounds have good anti-inflammatory and analgesic activities and can reduce serum levels of uric acid and glucose. One compound in particular, 5-methyl-1-phenyl-2(1H)-pyridorie, has the best activity and low toxicity.

EP 1138329A described an effective antifibrotic compound: the pirfenidone (PFD, 5-methyl-10phenyl-2(1H)-pyridone) as. U.S. patents 5,518,729 and 5,716,632 extend the anti-fibrotic activities to additional 44 compounds of either N-substituted 2(1H)-pyridone (I) or N-substituted 3(1H)-pyridone.

The efficacy of anti-fibrotic activity of 5-methyl-1-phenyl-2(1H)-pyridone (PIRFENIDONE, PFD) has been further demonstrated in various animal models and human clinical trials (Shimizu et al., Pirfenidone prevents collagen accumulation in the remnant kidney in rats with partial nephrectomy. Kidney Int. 52(Suppl 63):5239-243 (1997); Raghu et al., Treatment of idiopathic pulmonary fibrosis with a new antifibrotic agent, pirfenidone. Am. J. Respir. Crit. Care Med. 159:1061-1069 (1999)). These studies indicated that PIRFENIDONE not only prevents but also reverses the accumulation of excess extracellular matrix. The pharmacological mechanism of PIRFENIDONE has not been fully understood yet, but data to date indicate that PIRFENIDONE is an effective compound to down-regulate cytokines (including TGF-β), and decrease the activity of fibroblasts through regulating multiple factors.

A Chinese patent ZL02114190.8 described the identification and synthesis of total 38 new 5-methyl-1-(substituted phenyl)-2(1H)-pyridone compounds, having the following general structural formula (I):

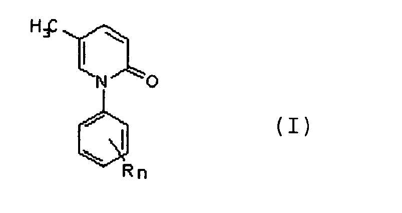

Where n=1, the substituent R can be F-Br-I. Where n=2, the substituent R can be F-Cl-Br-I'alkyl, alkoxy, halogenated alkyl group. The relative position for R groups is *ortho* or *meta* or *para*.

U.S. patent 5,716,632 listed 6 structural formulas of 5-methyl-1-(substituted phenyl)-2(1H)-pyridone originally described by Gadekar in U.S. patent 3,974,281. For these "substituted phenyl" compounds, Gadekar established a structure-activity relationship that teaches non-substituted phenyl as the compound with the best biological activities. However, it has been reported that a 5-methyl-1-(substituted phenyl)-2(1H)-pyridone, 1-(3'-fluorophenyl)-5-methyl-2(1H)-pyridone, displayed certain biological activities in vitro (J. Cent. South Univ. Med. Sci. 29:139 (2004)). Thus, it is of interest to determine whether substituted phenyl compounds having structural formula II would have any desirable anti-fibrotic activity.

### Description of the Invention

Based on the aforementioned background art, the present invention publishes a novel group of pyridone compounds, which can be used for the preparation of anti-fibrotic drugs for prevention and treatment of organ or tissue fibrosis.

The present invention provides a general structural formula (I) for the novel group of pyridone compounds: 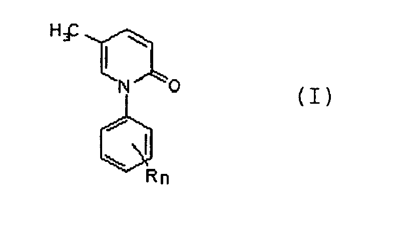 Where n=1, the substituent R can be F-Cl-Br-I-nitro-alkyl, alkoxy, halogenated alkyl group; where n=2, the substituent R can be F-Cl-Br-I'alkyl, alkoxy, halogenated alkyl group. These compounds can be used for the preparation of drug, which has a broad spectrum of anti-organ or anti-tissue fibrosis.

In the present invention, the term "anti organ or tissue fibrosis" means to prevent the fibrosis in organs/ tissues, or to slow or stop the fibrotic process in organs/ tissues, and/or to reverse the fibrotic condition in organs/ tissues.

In the structure formula (I), the preferred n number is 1 and the preferred R is F, Br, I.

Where in n = 2, R can be F, Cl, Br, I, saturated straight-chain alkyl group, saturated straight-chain alkoxy group, saturated straight-chain halogenated alkyl group. The relative position for R groups is *ortho* or *meta* or *para.*

The preferred position for F is *meta* position i.e.3'-fluorophenyl.

The alkyl substituent in the present invention is either saturated straight-chain or branch with 1-6 carbons, the preferred is 1-4 carbons.

Other examples of 5-methyl-1-(substituted phenyl)-2(1H)-pyridone (structural formula I) include, but are not limited to, the following compounds:

when n=1' R =Br, the compounds can be 1-(2-Bromophenyl)-5-methyl-2-(1H)-pyridone, 1-(3-Bromophenyl)-5-methyl-2-(1H)-pyridone, 1-(4-Bromophenyl)-5-methyl-2-(1H)-pyridone.

When n=1'R =F, the compounds can be 1-(2-Fluorophenyl)-5-methyl-2-(1H)-pyridone, 1-(3-Fluorophenyl)-5-methyl-2-(1H)-pyridone, 1-(4-Fluorophenyl)-5-methyl-2-(1H)-pyridone.

When n=1' R = I, the compounds can be 1-(2-Iodophenyl)-5-methyl-2-(1H)-pyridone, 1-(3-Iodophenyl)-5-methyl-2-(1H)-pyridone, 1-(4-Iodophenyl)-5-methyl-2-(1H)-pyridone.

When n=2' R =F, Br or Br, the compounds can be 1- (2, 3-Dibromophenyl)-5-methyl-2-(1H)pyridone, 1-(2,4-Dibromophenyl)-5-methyl-2-(1H)pyridone, 1-(2,5-Dibromophenyl)-5-methyl-2-(1H)pyridone, 1-(2,6-Dibromophenyl)-5-methyl-2-(1H)pyridone, 1-(3,4-Dibromophenyl)-5-methyl-2-(1H)pyridone, 1-(3,5-Dibromophenyl)-5-methyl-2-(1H)pyridone, 1-(2,3-Dichlorophenyl)-5-methyl-2-(1H)pyridone, 1-(2,4-Dichlorophenyl)-5-methyl-2-(1H)pyridone, 1-(2,5-Dichlorophenyl)-5-methyl-2-(1H)pyridone, 1-(2,6-Dichlorophenyl)-5-methyl-2-(1H)pyridone, 1-(3,5-Dichlorophenyl)-5-methyl-2-(1H)pyridone, 1-(2,3-Difluorophenyl)-5-methyl-2-(1H)pyridone, 1-(2,4-Difluorophenyl)-5-methyl-2-(1H)pyridone, 1-(2,5-Difluorophenyl)-5-methyl-2-(1H)pyridone, 1-(2,6-Difluorophenyl)-5-methyl-2-(1H)pyridone, 1-(3,5-Difluorophenyl)-5-methyl-2-(1H)pyridone,

When n=1 or 2 and R =trifluoromethyl, the compounds can be 1-(2-Trifluoromethylphenyl)-5-methyl-2-(1H)pyridone, 1-(4-Trifluoromethylphenyl)-5-methyl-2-(1H)pyridone, 1-(2,3-bistrifluoromethylphenyl)-5-methyl-2-(1H)pyridone, 1-(2,4- bistrifluoromethylphenyl)-5-methyl-2-(1H)pyridone, 1-(2,5- bistrifluoromethylphenyl)-5-methyl-2-(1H)pyridone, 1-(2,6- bistrifluoromethylphenyl)-5-methyl-2-(1H)pyridone, 1-(3,4- bistrifluoromethylphenyl)-5-methyl-2-(1H)pyridone, 1-(3,5- bistrifluoromethylphenyl)-5-methyl-2-(1H)pyridone.

When n =1 or 2 and R =methyl, the 5-methyl-1-(substitutedphenyl)-2-(1H)-pyridone are: 1-(2-methylphenyl)-5-methyl-2-(1H)pyridine, 1-(3-methylphenyl)-5-methyl-2-(1H)pyridine, 1-(2,3-Dimethylphenyl)-5-methyl-2-(1H)pyridine, 1-(2,4-Dimethylphenyl)-5-methyl-2-(1H)pyridine, 1-(2,5-Dimethylphenyl)-5-methyl-2-(1H)pyridine, 1-(2,6-Dimethylphenyl)-5-methyl-2-(1H)pyridine, 1-(3,4-Dimethylphenyl)-5-methyl-2-(1H)pyridine, 1-(3,5-Dimethylphenyl)-5-methyl-2-(1H)pyridine.

When n =1 or 2 and R =methoxyl, the compounds can be 1-(2-methoxyphenyl)-5-methyl-2-(1H)pyridine, 1-(3-methoxyphenyl)-5-methyl-2-(1H)pyridine, 1-(2,3-Dimethoxyphenyl)-5-methyl-2-(1H)pyridine, 1-(2,4-Dimethoxyphenyl)-5-methyl-2-(1H)pyridine, 1-(2,5-Dimethoxyphenyl)-5-methyl-2-(1H)pyridine, 1-(2,6-Dimethoxyphenyl)-5-methyl-2-(1H)pyridine, 1-(3,4-Dimethoxyphenyl)-5-methyl-2-(1H)pyridine, 1-(3,5-Dimethoxyphenyl)-5-methyl-2-(1H)pyridine.

According to the examples of the present invention, the preferred pharmaceutical compositions can be used as anti renal interstitial fibrosis, anti-glomerulus sclerosis, anti liver sclerosis, anti-pulmonary sclerosis, anti-peritoneal fibrosis, anti-myocardiac fibrosis, anti-skin fibrosis, anti-post-surgical adhesion, anti-benign prostate hypertrophy, anti-musculoskeletal fibrosis, anti-scleroderma, anti-Alzheimer's disease and anti-fibrotic vascular disease drug.

Also according to the examples of the present invention, the compounds of general structural formula (I) can be used to prepare therapeutic agent for treatment of liver fibrosis caused by schistosomiasis.

Also according to the examples of the present invention, the compounds of general structural formula (I) can be used to prepare therapeutic agent for treatment of glomerulus sclerosis caused by diabetes.
Also according to the examples of the present invention, the compounds of general structural formula (I) can be used to prepare therapeutic agent for treatment of renal interstitial fibrosis caused by blockade of urine track or diabetes.

Also according to the examples of the present invention, the prepared therapeutic agents from the compounds of general structural formula (I) can be administrated through oral, injectable or topical routes. The therapeutic agents can be prepared into any appropriated pharmaceutical formulations.

In the present invention, the selected compounds have been tested using ECM producing cells, and various animal models for fibrosis disease. The results demonstrate that 5-methyl-1-(substituted phenyl)-2-(1H)-pyridones are capable to suppress various ECM producing cells, and more potent than pirfenidone. Therefore, this group of novel compounds can be used as active ingredients for anti organ or tissues fibrotic drugs.

### Examples

In the present invention, 5-methyl-1-(3'-florourphynel)-2-(1H) -pyridone (AKF-PD) is used as an example to illustrate the preparation of antifibrotic drugs which include 5-methyl-1-(substituted phenyl)2-(1H)-pyridones. AKF-PD, 5-methyl-1-(3'-florourphynel)-2-(1H)-pyridone is made according to process described in Chinese patent ZL02114190.8.

### EXAMPLE 1

### Suppression of Mouse Mesangium Cell Proliferation by 1-(3'-Florourphynel)-5-Methyl-2-(1H)-Pyridone and PIRFENIDONE

Cell proliferation was measured by MTT assay. DMEM with 10% fetal serum was used as cell culture medium. The cells were prepared in suspension (1×10⁵/ml), and 100 µL of the suspension was transferred to each well of a 96 wells plate. Once the cells were attached to the plastic, the culture was changed to serum free medium and continued for another 24 hours. The serum free medium was aspirated, and various concentrations of 5-methyl-1-(3'-florourphynel)-2-(1H)-pyridone (AKD-PD) or PIRFENIDONE (PFD) in 10% serum medium were added into each well with 5 replicates for each concentration. The cells were stained with MTT (10 µL per well) at 12, 24, and 48 hours post drug treatment. After 4 hrs incubation, the medium with MTT was aspirated from each well. One hundred µL MTT solvent was added to each well for 15 min. The dissolved MTT was then measured with a plate reader at 570 nm.

The results are shown in Table 1. At 24 hours, 500 µg/ml of AKF-PD suppressed proliferation of mouse mesangium cells, but not 500 µg/ml of PIRFENIDONE. At the concentrations of 1000 µg/ml and 2500 µg/ml, both AKF-PD and PIRFENIDONE suppressed proliferation of mouse mesangium cells, but AKF-PD was more potent than pirfenidone. At the 48 hour time point, both AKF-PD and PIRFENIDONE had suppressive effect on mouse mesangium cells at all testing concentration levels; however, AKF-PD showed stronger effect than pirfenidone at 100 µg/ml, 500 µg/ml, 1000 µg/ml. In conclusion, AKF-PD can suppress proliferation of mouse mesangium cells and has more potent suppressive effects than PIRFENIDONE at certain concentration levels.

**TABLE 1**

| **Effects of AKF-PD and PFD on Mouse Mesangium Cells** | | | |
|---|---|---|---|
| Group | Optical Density at 570 nm | | |
| | 12 h | 24 h | 48 h |
| Control | 0.363±0.002 | 0.591±0.002 | 0.851±0.009 |
| AKF-PD 100 µg/ml | 0.368±0.003 | 0.594±0.003 | 0.812±0.002* |
| AKF-PD 500 µg/ml | 0.363±0.002 | 0.579±0.001* | 0.675±0.006** |
| AKF-PD 1000 µg/ml | 0.361±0.002 | 0.557±0.002** | 0.583±0.005*** |
| AKF-PD 2500 µg/ml | 0.355±0.002 | 0.541±0.002*** | 0.552±0.004*** |
| PFD 100 µg/ml | 0.362±0.003 | 0.591±0.002 | 0.830±0.003*⁺ |
| PFD 500 µg/ml | 0.360±0.003 | 0.590±0.001⁺⁺⁺ | 0.707±0.002**⁺⁺ |
| PFD 1000 µg/ml | 0.362±0.002 | 0.565±0.001***⁺⁺ | 0.599±0.004***⁺ |
| PFD 2500 µg/ml | 0.362±0.002⁺ | 0.548±0.002***⁺ | 0.559±0.-002*** |

| | | | |
|---|---|---|---|
| *p<0.05 vs control; **p<0.01 vs control; ***p<0.001 vs control +p<0.05 vs AKF-PD; ⁺⁺p<0.01 vs AKF-PD; ⁺⁺⁺p<0.001 vs AKF-PD | | | |

### EXAMPLE 2

### Suppression of Rat Myocardiac Fibroblasts Proliferation by -1-(3'-Florourphynel)- 5-Methyl 2-(1H)-Pyridone and PIRFENIDONE

Cell proliferation was measured by MTT assay. DMEM with 10% fetal serum was used as cell culture medium. The cells were prepared in suspension (1×10⁵/ml), and 100 µL of the suspension was transferred to each well of a 96 wells plate. Once the cells were attached to the plastic, the culture was changed to serum free medium and incubated for another 24 hours. Then, the serum free medium was aspirated, and various concentrations of 1-(3'-florourphynel)- 5-methyl-2-(1H)-pyridone (AKD-PD) or PIRFENIDONE (PFD) in 10% serum medium were added into each well with 5 replicates for each concentration. The cells were stained with MTT (10 µL per well) at 12, 24, or 48 hours post drug treatment. After 4 hrs incubation, the medium with MTT was aspirated from each well. One hundred µL MTT solvent was added to each well for 15 min, and the dissolved MTT was measured with a plate reader at 570 nm.

The results are shown in Table 2. At concentrations of 100 µg/ml, 500 µg/ml, 1000 µg/ml and 2500 µg/ml, both AFK-PD and PIRFENIDONE can suppress proliferation of rat myocardiac fibroblasts after 24 hours treatment; however, at 1000 µg/ml and 2500 µg/ml levels, AKF-PD was more potent than PIRFENIDONE. At the same concentration ranges, both AKF-PD and PIRFENIDONE showed suppressive effects on cell proliferation after 48 hours, but AKF-PD was more potent at 100 µg/ml, 500 µg/ml, and 1000 µg/ml. In conclusion, AKF-PD is a more potent anti-proliferation agent than PIRFENIDONE on rat myocardiac fibroblasts.

**TABLE 2**

| **Effects of AKF-PD and PFD on Rat Myocardiac Fibroblasts** | | | |
|---|---|---|---|
| Group | Optical Density at 570 nm | | |
| | 12 h | 24 h | 48 h |
| control | 0.330±0.002 | 0.445±0.016 | 0.684±0.008 |
| AKF-PD 100 µg/ml | 0.328±0.010 | 0.426±0.006* | 0.620±0.018*** |
| AKF-PD 500 µg/ml | 0.326±0.003 | 0.408±0.009** | 0.580±0.014*** |
| AKF-PD 1000 µg/ml | 0.332±0.006 | 0.392±0.008** | 0.538±0.009*** |
| AKF-PD 2500 µg/ml | 0.325±0.008 | 0.377±0.013*** | 0.514±0.005*** |
| PFD 100 µg/ml | 0.330±0.014 | 0.429±0.009* | 0.654±0.007*⁺ |
| PFD 500 µg/ml | 0.329±0.013 | 0.411±0.006* | 0.612±0.014***⁺⁺ |
| PFD 1000 µg/ml | 0.331±0.009 | 0.403±0.010**⁺ | 0.597±0.013***⁺⁺⁺ |
| PFD 2500 µg/ml | 0.329±0.008 | 0.392±0.009**⁺ | 0.566±0.027** |

| | | | |
|---|---|---|---|
| *p<0.05 vs control; **p<0.01 vs control; ***p<0.001 vs control ⁺p<0.05 vs AKF-PD; ⁺⁺p<0.01 vs AKF-PD; ⁺⁺⁺p<0.001 vs AKF-PD | | | |

### EXAMPLE 3

### Suppression of Human Stellate Cell Proliferation by 1-(3'-Florourphynel)-5-Methyl-2-(1H)-Pyridone and PIRFENIDONE

Cell proliferation was measured by MTT assay. DMEM with 10% fetal serum was used as cell culture medium. The cells were prepared in suspension (1×10⁵/ml), and 100 µL of the suspension was transferred to each well of a 96 wells plate. Once the cells were attached to the plastic, the culture was changed to serum free medium and incubated for another 24 hours. Then, the serum free medium was aspirated, and various concentrations of 1-(3'-florourphynel)-5-methyl-2-(1H)-pyridone (AKD-PD) or PIRFENIDONE (PFD) in 10% serum medium were added into each well with 5 replicates for each concentration. The cells were stained with MTT (10 µL per well) at 12, 24, or 48 hours post drug treatment. After 4 hrs incubation, the medium with MTT was aspirated from each well. One hundred µL MTT solvent was added to each well for 15 min, and the dissolved MTT was then measured with a plate reader at 570 nm.

The results are shown in Table 3. At 500 µg/ml, 1000 µg/ml, and 2500 µg/ml, both AKF-PD and PIRFENIDONE can suppress proliferation of human stellate cells beginning from 12 hours post drug treatment. At the 24 hours time point, 1000 µg/ml and 2500 µg/ml of AKF-PD was more suppressive than PIRFENIDONE. At 48 hours, AKF-PD was more suppressive than PIRFENIDONE at concentrations of 500 µg/ml, 1000 µg/ml, and 2500 µg/ml. In conclusion, AKF-PD is a more potent anti-proliferation agent than PIRFENIDONE on human stellate cells.

**TABLE 3**

| **Effects of AKF-PD and PFD on Human Stellate Cells** | | | |
|---|---|---|---|
| Group | Optical Density at 570 nm | | |
| | 12 h | 24 h | 48 h |
| Control | 0.207±0.001 | 0.370±0.002 | 0.455±0.002 |
| AKF-PD 100 µg/ml | 0.202±0.001 | 0.366±0.002 | 0.442±0.006 |
| AKF-PD 500 µg/ml | 0.202±0.001* | 0.341±0.003** | 0.406±0.002*** |
| AKF-PD 1000 µg/ml | 0.198±0.001** | 0.312±0.003*** | 0.385±0.004*** |
| AKF-PD 2500 µg/ml | 0.195±0.002** | 0.273±0.005*** | 0.246±0.001*** |
| PFD 100 µg/ml | 0.206±0.003 | 0.371±0.001 | 0.447±0.003 |
| PFD 500 µg/ml | 0.202±0.001* | 0.345±0.002** | 0.413±0.001***⁺⁺ |
| PFD 1000 µg/ml | 0.201±0.001* | 0.330±0.001***⁺⁺ | 0.402±0.001***⁺⁺ |
| PFD 2500 µg/ml | 0.198±0.001** | 0.278±0.001***⁺ | 0.306±0.002***⁺⁺⁺ |

| | | | |
|---|---|---|---|
| *p<0.05 vs control; **p<0.01 vs control; ***p<0.001 vs control ⁺p<0.05 vs AKF-PD; ⁺⁺p<0.01 vs AKF-PD; ⁺⁺⁺p<0.001 vs AKF-PD | | | |

### EXAMPLE 4

### Suppression of Rat Pulmonary Fibroblast Proliferation by 1-(3'-Florourphynel)-5-Methyl-2-(1H)-Pyridone and PIRFENIDONE

Cell proliferation was measured by MTT assay. DMEM with 10% fetal serum was used as cell culture medium. The cells were prepared in suspension (1×10⁵/ml), and 100 µL of the suspension was transferred to each well of a 96 wells plate. Once the cells were attached to the plastic, the culture was changed to serum free medium and incubated for another 24 hours. Then, the serum free medium was aspirated, and various concentrations of 1-(3'-florourphynel)-5-methyl-2-(1H)-pyridone (AKD-PD) or PIRFENIDONE (PFD) in 10% serum medium were added into each well with 5 replicates for each concentration. The cells were stained with MTT (10 µL per well) at 8, 20, or 44 hours post drug treatment. After 4 hrs incubation, the medium with MTT was aspirated from each well. One hundred µL MTT solvent was added to each well for 15 min, and the dissolved MTT was measured with a plate reader at 570 nm.

The results are shown in Table 4. After 24 hours treatment, AKF-PD could suppress proliferation of rat pulmonary fibroblasts at as low as 1000 µg/ml, and it was more suppressive than PIRFENIDONE at 2500 µg/ml. After 48 hours treatment, both compounds have effects at drug concentrations of 500 µg/ml, 1000 µg/ml, and 2500 µg/ml, but the anti-proliferation effects of AKF-PD were stronger than that of PIRFENIDONE at concentrations of 500 µg/ml and 1000 µg/ml. In conclusion, AKF-PD is a more potent anti-proliferation agent than PIRFENIDONE on rat pulmonary fibroblasts.

**TABLE 4**

| **Effects of AKF-PD and PFD on Rat Pulmonary Fibroblasts** | | | |
|---|---|---|---|
| Group | Optical Density at 570 nm | | |
| | 12 h | 24 h | 48 h |
| Control | 0.235±0.003 | 0.302±0.008 | 0.402±0.015 |
| AKF-PD 100 µg/ml | 0.235±0.009 | 0.301±0.013 | 0.399±0.008 |
| AKF-PD 500 µg/ml | 0.237±0.016 | 0.295±0.017 | 0.376±0.006* |
| AKF-PD 1000 µg/ml | 0.233±0.011 | 0.267±0.016** | 0.369±0.009** |
| AKF-PD 2500 µg/ml | 0.235±0.005 | 0.173±0.015*** | 0.241±0.008*** |
| PFD 100 µg/ml | 0.234±0.012 | 0.304±0.021 | 0.400±0.011 |
| PFD 500 µg/ml | 0.231±0.012 | 0.294±0.020 | 0.386±0.009**⁺⁺ |
| PFD 1000 µg/ml | 0.233±0.005 | 0.291±0.024⁺⁺ | 0.379±0.010 **⁺⁺⁺ |
| PFD 2500 µg/ml | 0.236±0.008 | 0.278±0.005**⁺⁺⁺ | 0.245±0.008^{***} |

| | | | |
|---|---|---|---|
| *p<0.05 vs control; **p<0.01 vs control; ***p<0.001 vs control ⁺p<0.05 vs AKF-PD; ⁺⁺p<0.01 vs AKF-PD; ⁺⁺⁺p<0.001 vs AKF-PD | | | |

### EXAMPLE 5

### Suppression of Human Skin Scar Forming Fibroblast Proliferation by 1-(3'-Florourphynel)-5-Methyl-2-(1H)-Pyridone and PIRFENIDONE

Cell proliferation was measured by MTT assay. DMEM with 10% fetal serum was used as cell culture medium. The cells were prepared in suspension (1×10⁵/ml), and 100 µL of the suspension was transferred to each well of a 96 wells plate. Once the cells were attached to the plastic, the culture was changed to serum free medium and incubated for another 24 hours. Then, the serum free medium was aspirated, and various concentrations of 1-(3'-florourphynel)-5-methyl-2-(1H)-pyridone (AKD-PD) or PIRFENIDONE (PFD) in 10% serum medium were added into each well with 5 replicates for each concentration. The cells were stained with MTT (10 µL per well) at 12, 24, or 48 hours post drug treatment. After 4 hrs incubation, the medium with MTT was aspirated from each well. One hundred µL MTT solvent was added to each well for 15 min, and the dissolved MTT was measured with a plate reader at 570 nm.

The results are shown in Table 5. After drug treatment for 24 hrs at concentrations of 100 µg/ml, 500 µg/ml, 1000 µg/ml, and 2500 µg/ml, both AKF-PD and PIRFENIDONE were capable of inhibiting the growth of human skin fibroblasts; however, AKF-PD was more potent than PIRFENIDONE at concentrations of 500 µg/ml, 1000 ug/ml, and 2500 µg/ml. After 48 hrs of treatment, 500 µg/ml or 1000 µg/ml of AKF-PD showed more inhibition than similar concentrations of PIRFEIDONE. In conclusion, AKF-PD is a more potent anti-proliferation agent than PIRFENIDONE on human skin fibroblasts.

**TABLE 5**

| **Effects of AKF-PD and PFD on Human Skin Scar Forming Fibroblasts** | | | |
|---|---|---|---|
| Group | Optical Density at 570 nm | | |
| | 12 h | 24 h | 48 h |
| Control | 0.195±0.008 | 0.263±0.005 | 0.381±0.001 |
| AKF-PD 100 µg/ml | 0.192±0.010 | 0.245±0.002* | 0.366±0.006^{*} |
| AKF-PD 500 µg/ml | 0.192±0.006 | 0.238±0.004* | 0.345±0.007^{*} |
| AKF-PD 1000 µg/ml | 0.192±0.009 | 0.221±0.004** | 0.323±0.009*** |
| AKF-PD 2500 µg/ml | 0.190±0.002 | 0.198±0.008*** | 0.267±0.001*** |
| PFD 100 µg/ml | 0.194±0.004 | 0.250±0.003* | 0.366±0.006* |
| PFD 500 µg/ml | 0.191±0.008 | 0.245±0.004*+ | 0.350±0.003***⁺⁺ |
| PFD 1000 µg/ml | 0.190±0.008 | 0.330±0.001*⁺⁺ | 0.328±0.004^{***++} |
| PFD 2500 µg/ml | 0.193±0.004 | 0.278±0.001***⁺⁺⁺ | 0.264±0.005*** |

| | | | |
|---|---|---|---|
| *p<0.05 vs control; **p<0.01 vs control; ***p<0.001 vs control ⁺p<0.05 vs AKF-PD; ⁺⁺p<0.01 vs AKF-PD; ⁺⁺⁺p<0.001 vs AKF-PD | | | |

### EXAMPLE 6

### Suppression of Human Peritoneal Mesothelial Cell Proliferation by 1-(3'-Florourphynel)-5-Methyl-2-(1H)-Pyridone and PIRFENIDONE

Cell proliferation was measured by MTT assay. DMEM with 10% fetal serum was used as cell culture medium. The cells were prepared in suspension (1×10⁵/ml), and 100 µL of the suspension was transferred to each well of a 96 wells plate. Once the cells were attached to the plastic, the culture was changed to serum free medium and incubated for another 24 hours. Then, the serum free medium was aspirated, and various concentrations of 1-(3'-florourphynel)-5-methyl-2-(1H)-pyridone (AKD-PD) or PIRFENIDONE (PFD) in 10% serum medium were added into each well with 5 replicates for each concentration. The cells were stained with MTT (10 µL per well) at 12, 24, or 48 hours post drug treatment. After 4 hrs incubation, the medium with MTT was aspirated from each well. One hundred µL MTT solvent was added to each well for 15 min, and the dissolved MTT was measured with a plate reader at 570 nm.

The results are shown in Table 6. After drug treatment for 24 hrs at concentrations of 500 µg/ml, 1000 µg/ml, and 2500 µg/ml, both AKF-PD and pirfenidone were capable of inhibiting the growth of human peritoneal mesothelial cells; however, AKF-PD was more potent than PIRFENIDONE at concentrations of 1000 µg/ml and 2500 µg/ml. For 48 hrs of treatment, 1000 µg/ml of AKF-PD showed more inhibition than similar concentration of PIRFEIDONE. In conclusion, AKF-PD is a more potent anti-proliferation agent than PIRFENIDONE on human peritoneal mesothelial cells.

**TABLE 6**

| **Effects of AKF-PD and PFD on Human Peritoneal Mesothelial Cells** | | | |
|---|---|---|---|
| Group | Optical Density at 570 nm | | |
| | 12 h | 24 h | 48 h |
| Control | 0.347±0.006 | 0.585±0.002 | 0.814±0.003 |
| AKF-PD 100 µg/ml | 0.344±0.004 | 0.583±0.004 | 0.807±0.007 |
| AKF-PD 500 µg/ml | 0.344±0.005 | 0.573±0.004* | 0.758±0.010^{*} |
| AKF-PD 1000 µg/ml | 0.343±0.004 | 0.553±0.004*** | 0.704±0.003*** |
| AKF-PD 2500 µg/ml | 0.346±0.005 | 0.502±0.003*** | 0.646±0.006^{***} |
| PFD 100 µg/ml | 0.346±0.006 | 0.584±0.005 | 0.810±0.006 |
| PFD 500 µg/ml | 0.343±0.004 | 0.577±0.003* | 0.766±0.004*** |
| PFD 1000 µg/ml | 0.363±0.003 | 0.563±0.003***⁺ | 0.714±0.002*** ⁺⁺⁺ |
| PFD 2500 µg/ml | 0.345±0.005 | 0.512±0.006***⁺ | 0.648±0.009*** |

| | | | |
|---|---|---|---|
| *p<0.05 vs control; **p<0.01 vs control; ***p<0.001 vs control ⁺p<0.05 vs AKF-PD; ⁺⁺p<0.01 vs AKF-PD; ⁺⁺⁺p<0.001 vs AKF-PD | | | |

### EXAMPLE 7

### Effects of 1-(3'-Florourphynel)-5-Methyl-2-(1H)-Pyridone (AKF-PD) on Glomerulosclerosis and Interstitial Renal Fibrosis

Rat diabetic kidney disease was induced by streptozotocin (STZ) to test the effectiveness of 1-(3'-florourphynel)-5-methyl-2-(1H)-pyridone (AKD-PD) in preventing anti-glomerulosclerosis and renal interstitial fibrosis.

Eight-weeks old male Wistar rat (180g~220g) were randomly grouped as normal, diabetic nephrosis, diabetic nephrosis/valsartan, or diabetic nephrosis/AKF-PD. Diabetes was induced by a single i.p. injection of STZ 55mg/kg. Diabetic condition was confirmed 24 hours later by the following test results: 13.9 mmol/L of fasting blood-glucose, 16.7 mmol/L random blood-glucose, and positive urine-glucose. If the diabetic rat had urine protein level higher than 30 mg/d 4 weeks later, a rat model was established for diabetic nephrosis.

Rats in the group of diabetic nephrosis/AKF-PD were orally fed 500mg/kg/d of AKF-PD, and rats in the group of diabetic nephrosis/valsartan were fed 30mg/kg/d of valsartan. Normal saline was fed to rats in the group of diabetic nephrosis. After 12 weeks of drug treatment, all rats were sacrificed and their kidneys were removed for pathological examination. The references for standard histopathological scoring system for glomerulus and renal tubules interstitial tissue are: Radford et al., Predicting renal outcome in IgA nephropathy. J. Am. Soc. Nephrol. 8:199-207 (1997); Zhao et al., Comparison of renoprotective effect between Angiotensin II receptor antagonist and angiotensin-converting enzyme inhibitor on puromycin nephropathy and their possible mechanism. Chin. J. Mult. Organ Dis. Elderly 1:36-40 (2002).

The results of microscopic examination of kidney tissues are shown in Table 7. Compared to diabetic nephrosis rats without any treatment, the AKF-PD-treated rats showed less damage on their glomerulus and renal tubules interstitial tissue, indicating that AKF-PD may effectively treat glomeruluslerosis and renal tubules interstitial fibrosis caused by diabetic condition.

**TABLE 7**

| **Histopathological Scores From Different Treatment Groups** | | | |
|---|---|---|---|
| Groups | Number of Animals | Glomerular Score | Renal Tubules Interstitial Tissue Score |
| Diabetic Nephrosis + AKF-PD | 5 | 1.29+-0.18 | 2.20+-0.14 |
| Diabetic Nephrosis + Valsartan | 8 | 1.36+-0.24 | 3.12+-0.64 |
| Diabetic Nephrosis | 9 | 1.63+-0.33 | 3.33+-0.54 |
| Normal Rat | 5 | 0.21+-0.04 | 0.48+-0.14 |

### EXAMPLE 8

### Effects of 1-(3'-Florourphynel)-5-Methyl-2-(1H)-Pyridone (AKF-PD) on Pulmonary Fibrosis

Bleomycin-induced rat pulmonary fibrosis is selected for testing 1-(3'-florourphynel)-5-methyl-2-(1H)-pyridone (AKD-PD). Male Sprague-Dawley rats (6-8 weeks old, 180g~220g) were cared under regular condition. The animals were randomly divided into 3 groups: sham surgical group, model disease group, and disease/AKF-PD group. 6 mg/kg/4ml of Bleomycine was slowly infused into the trachea of rats in the model disease and disease/AKF-PD groups. Same amount of normal saline was infused into the trachea of rats in the sham surgical group.

500 mg/kg AKF-PD was directly flashed into the stomach of rats in the disease/AKF-PD group daily from 2 days prior to operation through 27 days after operation. Normal saline was used for all animals in both disease and sham surgical groups. The animals were sarcrificed at 27 day post surgery and the lung tissues were removed for pathological sample preparation. The HE-stained lung tissues from each rat were examined under microscope to determine the frequency and severity of fibrotic lesion. Evaluation of lung tissue fibrosis was carried out according to the method of Szapiel et al., Bleomycin-induced interstitial pulmonary disease in the nude, athymic mouse. Am. Rev. Respir. Dis. 120:893-9 (1979). As shown in Table 8, rats treated with AKF-PD showed less severe fibrotic lesions as compared to those in the disease group, indicating AKF-PD may be an effective agent for treating pulmonary fibrosis disease.

**TABLE 8**

| **Comparison of Frequency and Severity of Fibrotic Lesions** | | | |
|---|---|---|---|
| Severity of Fibrosis | AKF-PD group 8 rats Total | Disease group 6 rats Total | Sham operation 7 rats Total |
| None | 0 | 0 | 2 |
| Milder | 6 | 1 | 5 |
| Moderate | 1 | 4 | 0 |
| Sever | 1 | 1 | 0 |

### EXAMPLE 9

### Effects of 1-(3'-Florourphynel)-5-Methyl-2-(1H)-Pyridone (AKF-PD) on Liver Fibrosis

Kun Ming (KM) mice were infected with *Schistosoma miracidium* to induce schistosomial liver fibrosis. Four to six weeks old male Kun Ming (KM) mice (18-22g) were randomly grouped into healthy, infected, infected/Pyquiton, infected/γ-interferon, and infected/AKF-PD groups. Ten *Schistosoma miracidium* were placed on shaved abdominal skin of each mouse for infection. Eight weeks post-infection, mice in the groups of infected/Pyquiton, infected/γ-interferon, and infected/AKF-PD were disinfected by treating with 650mg/kg Pyquiton for 4 days. Five hundred mg/kg AKF-PD were given daily orally to the disinfected mice of infected/AKF-PD group. The disinfected mice were given (i.m.) 50,000 unit γ-interferon daily. The disinfected mice in the infected/Pyquiton and infected groups were orally given normal saline once a day in the same way as administration of drug treatment. Drug or normal saline treatment was continued for 8 weeks. All mice were sacrificed one week after discontinue of drug treatment, and the left lobe of liver from each mouse was taken for pathological examination.

Examination of HE-stained slices of liver tissue were carried out as follows. In general, after 16 weeks of *Schistosoma miracidia* infection, the area of schistosomo ovum-induced nodule would directly correlate to the severity of liver fibrosis. Therefore, the area of schistosomo ovum-induced nodule was measured using a high-resolution, color pathology graphic analyzer (HPIAS-1000). The sum of area of 5 nodules with densely packed ovum and the sum of area of 5 nodules with lightly packed ovum were measured for each slice. As shown in Tables 9 and 10, AKF-PD-treated animals had smaller area (µm²) of schistosomo ovum nodule than those in either no-treatment (infected only) or Pyquiton-treated group. These results indicate that AKF-PD may be an effective pharmacological agent to treat schistosoma liver fibrosis.

**TABLE 9**

| **Comparison of Schistosomo Ovum-Induced Nodule Area** | | | | |
|---|---|---|---|---|
| | animal treatment | Number of animal | Mean (µm²) | S.D. |
| densely Packed Nodule | Infected/ γ-interferon, | 9 | 83706.79 | 22943.48 |
| | Infected/AKF-PD. | 10 | 80155.11 | 25419.82 |
| | Infected only | 10 | 111604.59 | 30115.49 |
| | Infected/Pyqui ton | 11 | 125823.35 | 31708.85 |
| lightly Packed Nodule | Infected/ y-interferon, | 9 | 32407.14 | 10078.30 |
| | Infected/AKF-PD. | 10 | 30266.68 | 11069.89 |
| | Infected only | 10 | 41116.13 | 11246.94 |
| | Infected/Pyqui ton | 11 | 45418.59 | 18001.53 |

**TABLE 10**

| ***P* values for Groups in Table 9** | | |
|---|---|---|
| Groups | Most ovum | Least ovum |
| Infected only vs infected/ y-interferon, | 0.038 | 0.095 |
| infected/ y-interferon, vs infected/ Pyquiton | 0.004 | 0.058 |
| Infected/ Pyquiton vs infected/AKF-PD. | 0.002 | 0.032 |
| Infected only vs infected/ Pyquiton | 0.306 | 0.516 |
| Infected only vs infected/AKF-PD. | 0.021 | 0.043 |
| Infected/ γ-interferon, vs infected/AKF-PD. | 0.754 | 0.666 |

### EXAMPLE 10

### Effects of 1-(3'-Florourphynel)-5-Methyl-2-(1H)-Pyridone (AKF-PD) in a Rat Model of Interstitial Naporoyl Fibrosis

Anti-fibrosis effects of AKF-PD was tested on a SD rat model for interstitial naphorial fibrosis induced by surgical ligation of single side ureter. Eight weeks old male SD rats (180g~220g) were randomly divided into sham surgical group, disease model group, Enalapril (10mg/kg/d) group and AKF-PD (500mg/kg/d) group. Under aseptic condition, all animals in the groups of disease model, Enalapril and AKF-PD had a surgical procedure for ligation of the left side ureter. The animals of the sham surgical group experienced the same surgical procedure except the ligation step. The respective drugs were administrated orally to rats in the groups of Enalapril and AKF-PD from one day prior to the procedure to 14 days post surgical procedure. Normal saline was administrated in a similar fashion to the rats in the groups of disease model and sham surgical. The animals were sacrificed 14 days after surgical procedure and their left kidney were removed for pathological (HE staining) examination. Histological scoring for interstitial compartment was done according to Radford's method ( Radford et al., Predicting renal outcome in IgA nephropathy. J. Am. Soc. Nephrol. 8:199-207 (1997)). As shown in Table 11, rats treated with AKF-PD showed reduced lesion on interstitial tissues comparing to those in the groups of disease model and Enalapril, indicating AKF-PD may be an effective drug for interstitial renal fibrosis.

**TABLE 11**

| **Comparison of Histological Scores for Interstitial Compartment** | | | | |
|---|---|---|---|---|
| Group | Sham surgical | Disease Model | Enalapril | AKF-PD |
| Number of rats (n) | 10 | 9 | 9 | 8 |
| Score | 1.33±0.58 | 10.24±0.99 | 9.21±0.64 | 6.43±1.28 |

### EXAMPLE 11

### Comparison of Acute Toxicity of 1-(3-fluorophenyl)-5-methyl-2(1H)-pyridone (AKF-PD) and PIRFENIDONE

Male and female Kun Ming (KM) mice weighing between 18g - 22g were acquired from the animal facility of Hsiang-Ya Medical College, the Central South University. Fifty Kun Ming mice were randomly assigned into 5 groups with 5 male and 5 female mice for each group. The animals went through fasting with a normal water supply before starting drug treatment (either AKF-PD or PIRFENIDONE). The drug was administrated orally by gavage. The volume of liquid drug was 20ml/kg body weight. The range of dosage for AKF-PD administrated was from 1071 mg/kg to 6000 mg/kg. The dosage difference between two adjacent doses was 1:0.65 (a dose vs the next lower dose). All animals were maintained under a routine condition. Acute toxic reaction and death within 14 days of post drug treatment were recorded. Autopsy was performed on all dead animals and visual examination was performed on all organs.

LD₅₀ was calculated according to Bliss method. Acute toxicity LD₅₀ for 1-(3-fluorophenyl)-5-methyl-2(1H)-pyridone was 2979.89 mg/kg with a 95% confidence limit of 2402.70 --3695.73 mg/kg (Table 12). LD₅₀ for PIRFENIDONE was 955.4 mg/kg with a 95% confidence limit of 550.9 -- 1656.7 mg/kg (Table 13). The results of Table 13 are very close to those reported in the literature: 997.7 mg/kg (U.S. patent 5,310,562) and 1112 mg/kg(Pharmaceutical Care and Research 5:4823 (2005)). These results indicate that the toxicity for AKF-PD is less than 3 fold compared to that of PIRFENIDONE (2978 vs 955).

**TABLE 12**

| **LD₅₀ of 5-methyl-1-(3-fluorophenyl)-2(1H)-pyridone** | | | | | |
|---|---|---|---|---|---|
| Dosage(mg /kg) | Dosage in log scale (x) | Number of Mice | Number of death | Percentage of death (%) | LD₅₀ and 95% confidence limit |
| 6000.0 | 3.7782 | 10 | 9 | 90 | |
| 3900.0 | 3.5911 | 10 | 7 | 70 | |
| 2535.0 | 3.4040 | 10 | 4 | 40 | 2979.89 mg/kg |
| 1647.8 | 3.2169 | 10 | 1 | 10 | (2402.70~ |
| 1071.0 | 3.0298 | 10 | 0 | 0 | 3695.73) |

**TABLE 13**

| **LD₅₀ of PIRFENIDONE** | | | | | |
|---|---|---|---|---|---|
| Dosage (mg/kg) | Dosage in log scale (x) | Number of Mice | Number of death | Percentage of death (%) | LD₅₀ and 95% confidence limit |
| 6000 | 3.778 | 10 | 10 | 100 | |
| 3900 | 3.591 | 10 | 10 | 100 | 955.4 mg/kg |
| 2535 | 3.030 | 10 | 8 | 80 | (550.9~ |
| 1647.8 | 3.404 | 10 | 6 | 60 | 1656.7) |
| 1071 | 3.217 | 10 | 5 | 50 | |

## Claims

1. Use of compounds, 5-methyl-1-(substituted phenyl)-2(1H)-pyridones, having general structure formula (I), 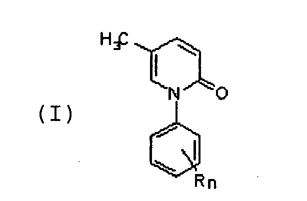 in the manufacture of medicaments for treating organ or tissue fibrosis. Wherein, when n=1, the substitution R = F, Cl, Br, I, nitro, alkyl group, alkoxy group, halogenated alkyl group; when n=2, the substitution R = F, C1, Br, I, alkyl group, alkoxy group, halogenated alkyl group.

2. The use of claim 1, wherein n = 1, then R can be F or Br or I; if n is 2 then R can be F or C1 or Br or I or saturated straight-chain alkyl group, or saturated straight-chain alkoxy group, or saturated straight-chain halogenated alkyl group, and the relative position for R groups can be *ortho* or *meta* or *para.*

3. The use of claim 2, wherein said the compounds, 5-methyl-1-(substituted phenyl)-2(1H)-pyridones (general structure formula I), having the following characteristics:
when n=1' R=Br' the compounds are: 1- (2-Bromophenyl)-5-methyl-2-(1H)-pyridone, 1-(3-Bromophenyl)-5-methyl-2-(1H)-pyridone, 1-(4-Bromophenyl)-5-methyl-2-(1H)pyridone.
when n=1' R=F' the compounds are: 1- (2-Fluorophenyl) -5-methyl-2-(1H)-pyridone, **1-(3-Fluorophenyl)-5-methyl-2-(1H)-pyridone,** 1-(4-Fluorophenyl)- 5-methyl-2-(1H)-pyridone.
when n=1' R= I' the compounds are: 1- (2-Iodophenyl) -5-methyl-2-(1H)-pyridone, 1-(3-Iodophenyl)-5-methyl-2-(1H)-pyridone, 1-4-Iodophenyl)-5-methyl-2-(1H)-pyridone.
When n=2' R=F or Br or Cl' the compounds are: 1-(2,3-Dibromophenyl)- 5-methyl-2-(1H)-pyridone, 1-(2,4-Dibromophenyl)- 5-methyl-2-(1H)-pyridone 1-(2,5-Dibromophenyl)- 5-methyl-2-(1H)-pyridone, 1-(2,6-Dibromophenyl)- 5-methyl-2-(1H)-pyridone, 1-(3,4-Dibromophenyl)- 5-methyl-2-(1H)-pyrido 1-(3,5-Dibromophenyl)- 5-methyl-2-(1H)-pyridone, 1-(2,3-Dichlorophenyl)- 5-methyl-2-(1H)-pyridone, 1-(2,4-Dichlorophenyl)- 5-methyl-2-(1H)-pyridone, 1-(2,5-Dichlorophenyl)- 5-methyl-2-(1H)-pyridone, 1-(2,6-Dichlorophenyl)- 5-methyl-2-(1H)-pyridone, 1-(3,5-Dichlorophenyl)- 5-methyl-2-(1H)-pyridone, 1-(2,3-Difluorophenyl)- 5-methyl-2-(1H)-pyridone, 1-(2,4-Difluorophenyl)- 5-methyl-2-(1H)-pyridone, 1-(2,5-Difluorophenyl)- 5-methyl-2-(1H)-pyridone, 1-(2,6-Difluorophenyl)- 5-methyl-2-(1H)-pyridone, 1-(3,5-Difluorophenyl)- 5-methyl-2-(1H)-pyridone.
When n=1 or 2' R=Trifluoromethyl, the compounds are: 5-methyl-1-(2-Trifluoromethylphenyl)-2-(1H)-pyridone, 5-methyl-1-(4-Trifluoromethylphenyl)-2-(1H)-pyridone, 5-methyl-1-(2,3-bis-trifluoromethylphenyl)-2-(1H)-pyridone, 5-methyl-1-(2,4-bis-itrifluoromethylphenyl)-2-(1H)-pyridone, 5-methyl-1-(2,5-bis-trifluoromethylphenyl)-2-(1H)-pyridone, 5-methyl-1-(2,6-bis-trifluoromethylphenyl)-2-(1H)-pyridone, 5-methyl-1-(3,4-bis-trifluoromethylphenyl)-2-(1H)-pyridone, and 5-methyl-1-(3,5-bis-trifluoromethylphenyl)-2-(1H)-pyridone.
When n =1 or 2 and R=methyl, the compounds are: 5-methyl-1-(2-methylphenyl)-2-(1H)-pyridone, 5-methyl-1-(3-methylphenyl)-2-(1H)-pyridone, 1-(2,3-Dimethylphenyl)-5-methyl-2-(1H)-pyridone 1-(2,4-Dimethylphenyl)-5-methyl-2-(1H)-pyridone, 1-(2,5-Dimethylphenyl)-5-methyl-2-(1H)-pyridone, 1-(2,6-Dimethylphenyl)-5-methyl-2-(1H)-pyridone, 1-(3,4-Dimethylphenyl)-5-methyl-2-(1H)-pyridone, and 1-(3,5-Dimethylphenyl)-5-methyl-2-(1H)-pyridone.
When n=1 or 2' R=methoxy, the compounds are: 5-methyl-1-(2-methoxyphenyl)-2-(1H)-pyridone, 5-methyl-1-(3-methoxyphenyl)- 2-(1H)-pyridone, 1-(2,3-Dimethoxyphenyl)-5-methyl-2-(1H)-pyridone, 1-(2,4-Dimethoxyphenyl)-5-methyl-2-(1H)-pyridone 1-(2,5-Dimethoxyphenyl)-5-methyl-2-(1H)-pyridone, 1-(2,6-Dimethoxyphenyl)-5-methyl-2-(1H)-pyridone, 1-(3,4-Dimethoxyphenyl)-5-methyl-2-(1H)-pyridone, and 1-(3,5-Dimethoxyphenyl)-5-methyl-2-(1H)-pyridone.

4. The use of claim 3, wherein the preferred substitution R is fluoro at position 3.

5. The use of claim 1 or 2, wherein the saturated straight-chain alkyl group or saturated branched-chain alkyl group has 1-6 carbons with preference of 1-4 carbons.

6. The use of claims 1-5 wherein said fibrosis including glomerulus sclerosis, renal interstitial fibrosis, liver fibrosis, pulmonary fibrosis, perodoneal fibrosis, myocardiac fibrosis, and fibrosis of skin, post-surgical adhesion, benign prostate hypertrophy, musculoskeletal fibrosis, scleroderma, Alzheimer's disease, fibrotic vascular disease, and glaucoma.

7. The use of claim 6, wherein said glomerulus sclerosis is caused by diabetic condition.

8. The use of claim 6, wherein said renal interstitial fibrosis is due to either blockade of the ureter or diabetic disease.

9. The use of claim 6, wherein said liver fibrosis is from the infection of schistosoma.

10. The use of claims 1-9, wherein the administration of the said compounds by oral, injection, or topical application; the said compounds can be formulated with compatible pharmaceutical carrier and/or other drugs.
